# EUROPEAN PATENT SPECIFICATION

(11) **EP 3 842 018 B1**
(45) Date of publication and mention of the grant of the patent: **15.11.2023**
(21) Application number: 19851683.3
(22) Date of filing: 21.08.2019
(51) Int. Cl.: A61F 13/536, A61F 13/472, A61F 13/475, A61F 13/511, A61F 13/533

(54) **ABSORBENT ARTICLE**
ABSORBIERENDER ARTIKEL
ARTICLE ABSORBANT

(30) Priority: 22.08.2018 JP 2018155524
(43) Date of publication of application: 30.06.2021
(73) Proprietor: DAIO PAPER CORPORATION, Shikokuchuo-shi Ehime 799-0492 (JP)
(72) Inventor: WATANABE Ikuya, Sakura-shi, Tochigi 329-1411 (JP)
(74) Representative: Bandpay & Greuter
(86) International application number: PCT/JP2019/032642
(87) International publication number: WO 2020/040199

(56) References cited:
- JP-A- H10 155 832
- JP-A- H10 155 832
- JP-A- 2003 230 592
- JP-A- 2003 230 592
- JP-A- 2007 152 032
- JP-A- 2007 152 032
- JP-A- 2012 050 497
- JP-A- 2012 050 497
- JP-A- 2016 010 633
- JP-A- 2016 010 633
- JP-A- 2018 051 110
- JP-A- 2018 051 110
- JP-U- 3 204 425
- JP-U- 3 204 425

## Description

### Technical Field

The present invention mainly relates to an absorbent article such as a sanitary napkin, a vaginal discharge sheet, an incontinence pad, or toiletries, and specifically relates to an absorbent article in which a compressed groove that is recessed toward a non-skin side is formed at a rear portion of a skin contact surface.

### Background Art

Conventionally, as the absorbent article, there has been a known absorbent article in which an absorber made of fluff pulp, etc. is interposed between a liquid-impermeable back sheet such as a polyethylene sheet or a polyethylene sheet laminated nonwoven fabric and a liquid-permeable face sheet such as a nonwoven fabric or a liquid-permeable plastic sheet.

A lot of improvements have been made to this type of absorbent article, and in order to prevent the leakage of bodily fluid, various forms of compressed grooves have been developed. For example, Patent Document 1 below discloses an absorbent article in which a first flexible shaft and a second flexible shaft are formed to extend in a vertical direction at an intervals at positions apart from a center line extending in the vertical direction on the right and left sides, and a pair of lateral flexible shafts connecting between first flexible shafts are formed at an interval in the vertical direction. In addition, Patent Document 2 below discloses that a plurality of rear compressed grooves is provided in a longitudinal direction. Patent Document D3 relates to an absorbent article.

### Citation List

### Patent Document

Patent Document 1: Japanese Patent No. 4,180,865
Patent Document 2: Japanese Patent No. 5,543,847
Patent Document 3: JP2016010633

### Summary of the Invention

### Technical Problem

In the absorbent articles described in Patent Documents 1 and 2, the compressed groove serves as a flexible shaft and is easily deformed along a shape of a body, and at the same time, bodily fluid is blocked by the compressed groove, so that it is considered that the effect that diffusion of bodily fluid is suppressed is also achieved.

However, in the absorbent article described in Patent Document 1, since a central absorbent portion is surrounded by the first flexible shafts extending in the vertical direction and the lateral flexible shafts connecting between the first flexible shafts, there is a risk of leakage at an overflowing part when the bodily fluid absorbed and saturated by the central absorbent portion overflows from the surrounding first flexible shafts and a part of the lateral flexible shafts.

In addition, in the absorbent article described in Patent Document 2, even though a plurality of rear compressed grooves having a shape bulging toward the rear side is arranged at intervals in the longitudinal direction in a rear portion, there is a risk of leakage from the side when the bodily fluid blocked by the rear compressed grooves overflows from a region surrounded by the rear compressed grooves to both sides.

As described above, the conventional absorbent articles may not have sufficient measures particularly against the rear leakage, and there is room for further improvement.

Therefore, a main object of the invention is to provide an absorbent article in which rear leakage is reliably prevented.

### Solution to Problem

To solve the above-mentioned problem, as the invention according to claim 1, provided is an absorbent article in which an absorber is interposed between a face sheet and a back sheet, the absorbent article including
a plurality of widthwise compressed grooves which is disposed in a region on a rear side of a region corresponding to a bodily fluid discharge part of a wearer, extends along substantially a width direction of the absorbent article, is formed in shapes bulging toward a rear side, and is arranged at intervals in a longitudinal direction of the absorbent article, and a pair of right and left longitudinally compressed grooves which extends along substantially a longitudinal direction of the absorbent article and is arranged on outer sides in the width direction at a predetermined separating distance from the widthwise compressed grooves, respectively.

In the region on the rear side of the region corresponding to the bodily fluid discharge part of the wearer, the widthwise compressed grooves extending along substantially the width direction of the absorbent article and the longitudinally compressed grooves extending along substantially the longitudinal direction of the absorbent article are provided in predetermined forms, respectively. The plurality of widthwise compressed grooves is formed in shapes bulging toward the rear side and arranged at intervals in the longitudinal direction of the absorbent article. For this reason, the bodily fluid diffusing toward the rear side can be blocked by the plurality of widthwise compressed grooves in stages and absorbed into the absorber. In addition, since the longitudinally compressed grooves are arranged on the respective outer sides in the width direction at the predetermined separating distance from the widthwise compressed groove, the bodily fluid blocked by the widthwise compressed groove can be prevented from diffusing outward in the width direction and leaking laterally. Moreover, since a separation portion is provided between the widthwise compressed groove and the longitudinally compressed groove, the bodily fluid blocked by the widthwise compressed groove and saturated in this region diffuses to the rear side through the separation portion, so that the bodily fluid can be further absorbed into the rear side region. Therefore, the rear leakage can be reliably prevented.

Groove widths of the widthwise compressed grooves gradually increase so that one arranged relatively on the rear side of the absorbent article has a larger groove width.

The plurality of widthwise compressed grooves arranged at intervals in the longitudinal direction of the absorbent article, one arranged relatively on the rear side of the absorbent article has a gradually larger groove width. Therefore, the widthwise compressed groove arranged on the rear side has a higher effect of blocking the bodily fluid, so that the rear leakage of the bodily fluid can be more reliably prevented.

Lengths of the widthwise compressed grooves gradually may increase so that one arranged relatively on the rear side of the absorbent article has a larger length.

Of the plurality of widthwise compressed grooves arranged at intervals in the longitudinal direction of the absorbent article, one arranged relatively on the rear side of the absorbent article may have a gradually larger length. Therefore, the widthwise compressed groove arranged on the rear side may have a higher effect of blocking the bodily fluid, so that the rear leakage of the bodily fluid can be more reliably prevented.

The longitudinally compressed grooves may form in shapes bulging outward in the width direction of the absorbent article.

Since the longitudinally compressed grooves may form in shapes bulging outward in the width direction of the absorbent article, the bodily fluid may be easily retained inside the longitudinally compressed grooves, and leakage from the side portion can be more reliably prevented.

Separating distances between the widthwise compressed grooves and the longitudinally compressed grooves gradually decrease so that one arranged relatively on the rear side of the absorbent article has a smaller separating distance.

Separating distances between the widthwise compressed grooves and the longitudinally compressed grooves gradually decrease so that one arranged relatively on the rear side of the absorbent article has a smaller separating distance. Thus, in a widthwise compressed groove arranged near the front side, more bodily fluid can diffuse to the rear side through the separation portion with respect to the longitudinally compressed groove, and it is possible to prevent the bodily fluid blocked by the widthwise compressed groove arranged near the front side from overflowing and leaking to the side. Further, as the amount of bodily fluid gradually diffusing toward the rear side decreases as the bodily fluid is absorbed and retained by the absorber, the separation portion is narrowed. Thus, the bodily fluid blocked by the widthwise compressed groove can be more reliably absorbed by the absorber in a region surrounded by the widthwise compressed groove and the longitudinally compressed grooves on both sides.

A middle-height portion of the absorber thickened toward a skin side may be formed in a section including the region corresponding to the bodily fluid discharge part of the wearer, and a rear end of the middle-height portion is located on a rear side of front ends of the longitudinally compressed grooves.

Since the middle-height portion is formed in the section including the region corresponding to the bodily fluid discharge part of the wearer, and the rear end of the middle-height portion may be located on the rear side of the front ends of the longitudinally compressed grooves, the bodily fluid absorbed in the middle-height portion may diffuse from the rear end of the middle-height portion into a region in which the longitudinally compressed grooves are arranged on both sides, and it may become easy to reliably absorb the bodily fluid into the absorber while preventing leakage of the bodily fluid by the longitudinally compressed grooves and the widthwise compressed grooves.

A pair of right and left central longitudinally compressed grooves extending along substantially the longitudinal direction of the absorbent article may be provided on both sides of a region including the region corresponding to the bodily fluid discharge part of the wearer, and a right-left separating distance at rear ends of the central longitudinally compressed grooves is smaller than a right-left separating distance at front ends of the longitudinally compressed grooves.

ln the case where the pair of right and left central longitudinally compressed grooves may be provided on both sides of the region including the region corresponding to the bodily fluid discharge part of the wearer, in order to prevent leakage due to the bodily fluid diffusing to the rear side along the central longitudinally compressed groove, the right-left separating distance at the rear ends of the central longitudinally compressed grooves may be set smaller than the right-left separating distance at the front ends of the longitudinally compressed grooves. In this way, the bodily fluid diffusing to the rear side along the central longitudinally compressed grooves may diffuse into the region in which the longitudinally compressed grooves are arranged on both sides, and it may become easy to reliably absorb the bodily fluid into the absorber while preventing leakage of the bodily fluid by the longitudinally compressed grooves and the widthwise compressed grooves.

### Advantageous Effects of the Invention

As described above in detail, according to the invention, it is possible to provide an absorbent article in which rear leakage is reliably prevented.

### Brief Description of the Drawings

Fig. 1 is a partially broken development view of a sanitary napkin 1 according to the invention.
Fig. 2 is a diagram taken along line II-II of Fig. 1.
Fig. 3 is a diagram taken along line III-III of Fig. 1.
Fig. 4 is a development view of the sanitary napkin 1 illustrating a diffusion state of bodily fluid.
Fig. 5 is a plan view (No. 1) illustrating a compressed groove on a rear side according to another embodiment.
Fig. 6 is a plan view (No. 2) illustrating a compressed groove on a rear side according to another embodiment.

### Mode for Carrying Out the Invention

Hereinafter, embodiments of the invention will be described in detail with reference to the drawings.

### [Basic structure of sanitary napkin 1]

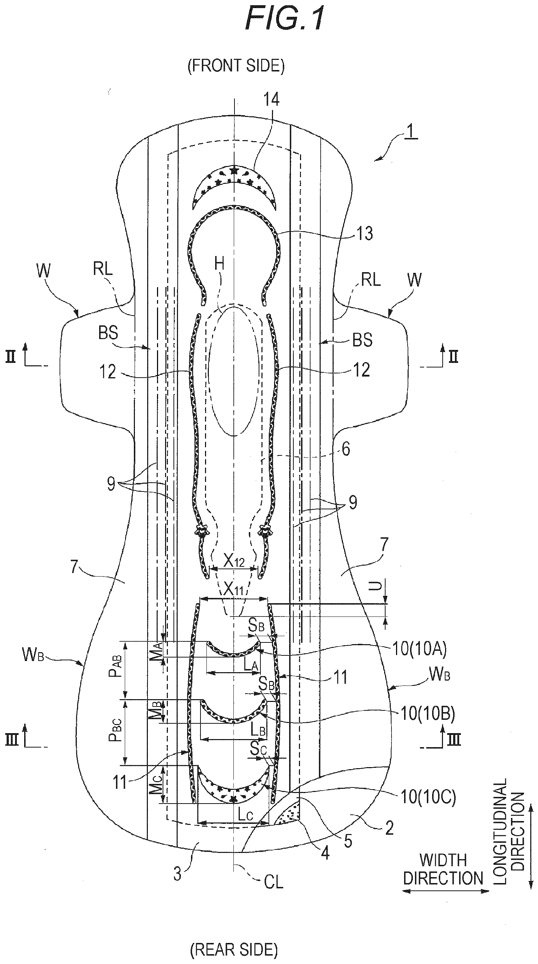

As illustrated in Figs. 1 to 3, a sanitary napkin 1 of the invention includes a liquid-impermeable back sheet 2 made of a polyethylene sheet, etc., a liquid-permeable face sheet 3 that allows rapid permeation of menstrual blood, vaginal discharge, etc. (hereinafter collectively referred to as bodily fluid), etc., an absorber 4 made of fluff pulp or synthetic pulp interposed between these sheets 2 and 3, and side nonwoven fabrics 7 and 7 which are provided in both side portions on a skin contact surface side over almost the entire length in a longitudinal direction, use a substantially side edge portion of the absorber 4 as a standing base edge, and form a pair of right and left three-dimensional gathers BS and BS provided to protrude to a skin side in a predetermined section in the longitudinal direction of the napkin including at least a region corresponding to a bodily fluid discharge part H of a wearer. Around the absorber 4, in upper and lower end edge portions thereof, the liquid-impermeable back sheet 2 and the liquid-permeable face sheet 3 are joined at outer edge portions by an adhesive such as hot melt or joining means such as heat sealing or ultrasonic sealing, and in both side edge portions thereof, the liquid-impermeable back sheet 2 extending laterally from the absorber 4 and the side nonwoven fabric 7 are joined by an adhesive such as hot melt or joining means such as heat sealing or ultrasonic sealing. A pair of right and left wing-shaped flaps W and W is formed at side positions of the absorber substantially corresponding to the bodily fluid discharge part H, and rear flaps W_{B} and W_{B} extending outward are formed in both side portions of a rear portion on a rear side (gluteal region side) thereof. Note that in the illustrated example, in order to maintain the shape of the absorber 4 and improve diffusibility, the absorber 4 is surrounded by an encapsulation sheet 5 made of crepe paper or nonwoven fabric. However, the encapsulation sheet 5 may not be provided. In addition, although not illustrated, a second sheet made of a hydrophilic nonwoven fabric, etc. having substantially the same shape as that of the liquid-permeable face sheet 3 may be arranged adjacent to the non-skin side of the liquid-permeable face sheet 3.

Hereinafter, the structure of the sanitary napkin 1 will be further described in more detail.

The liquid-impermeable back sheet 2 is made of a sheet material having at least water-blocking property such as polyethylene. However, from a viewpoint of preventing stuffiness, it is desirable to use a material having moisture permeability. As this water-blocking/moisture-permeable sheet material, a microporous sheet obtained by melt-kneading an inorganic filler in an olefin resin such as polyethylene or polypropylene to form a sheet, and then stretching the sheet in a uniaxial or biaxial direction is preferably used. On a non-skin side surface (outer surface) of the liquid-impermeable back sheet 2, one or a plurality of adhesive layers (not illustrated) is formed along the longitudinal direction of the napkin, so that the sanitary napkin 1 is fixed to underwear when worn on a body. As the liquid-impermeable back sheet 2, a polylaminated nonwoven fabric obtained by laminating a plastic film and a nonwoven fabric may be used.

Next, as the liquid-permeable face sheet 3, a perforated or non-perforated nonwoven fabric, a porous plastic sheet, etc. is preferably used. As a material fiber included in the nonwoven fabric, in addition to synthetic fibers such as olefins such as polyethylene and polypropylene, polyesters, and polyamides, it is possible to use recycled fibers such as rayon and cupra, and natural fibers such as cotton, and it is possible to use a nonwoven fabric obtained by an appropriate processing method such as a spunlace method, a spunbond method, a thermal bond method, a meltblown method, or a needle punch method. Among these processing methods, the spunlace method is excellent in flexibility and drapability, and the thermal bond method is excellent in bulkiness and high compression recovery. When a large number of through holes are formed in the liquid-permeable face sheet 3, the bodily fluid is rapidly absorbed, and a dry touch property becomes excellent. The fibers of the nonwoven fabric may be either long fibers or short fibers, and preferably short fibers are used in order to give the texture of a towel cloth. Further, in order to facilitate an embossing treatment, it is preferable to use an olefin fiber having a relatively low melting point such as polyethylene or polypropylene. Further, it is possible to preferably use a core-sheath fiber having a high melting point fiber as a core and a low melting point fiber as a sheath, a side-by-side type fiber, or a composite fiber of a split type fiber.

The absorber 4 interposed between the liquid-impermeable back sheet 2 and the liquid-permeable face sheet 3 is made of, for example, fluff pulp and a water-absorbent polymer. The water-absorbent polymer is mixed in the pulp included in the absorber, for example, as granular powder. Examples of the pulp include chemical pulp obtained from wood and pulp such as dissolving pulp containing cellulose fibers or artificial cellulose fibers such as rayon and acetate. Softwood pulp having a long fiber length is more preferably used than hardwood pulp in terms of function and cost. A basis weight of the absorber 4 may be set to 300 to 750 g/m², and preferably 300 to 400 g/m².

Further, the absorber 4 may be mixed with a synthetic fiber. As the synthetic fiber, for example, it is possible to use a polyolefin-based material such as polyethylene or polypropylene, a polyester-based material such as polyethylene terephthalate or polybutylene terephthalate, a polyamide-based material such as nylon, or a copolymer thereof, and it is possible to use a mixture of two of these types. In addition, it is possible to use a core-sheath fiber having a high melting point fiber as a core and a low melting point fiber as a sheath, a side-by-side type fiber, or a composite fiber such as a split type fiber. In the case where the synthetic fiber is a hydrophobic fiber, it is desirable to use the synthetic fiber which is surface-treated using a hydrophilizing agent to have an affinity for bodily fluid.

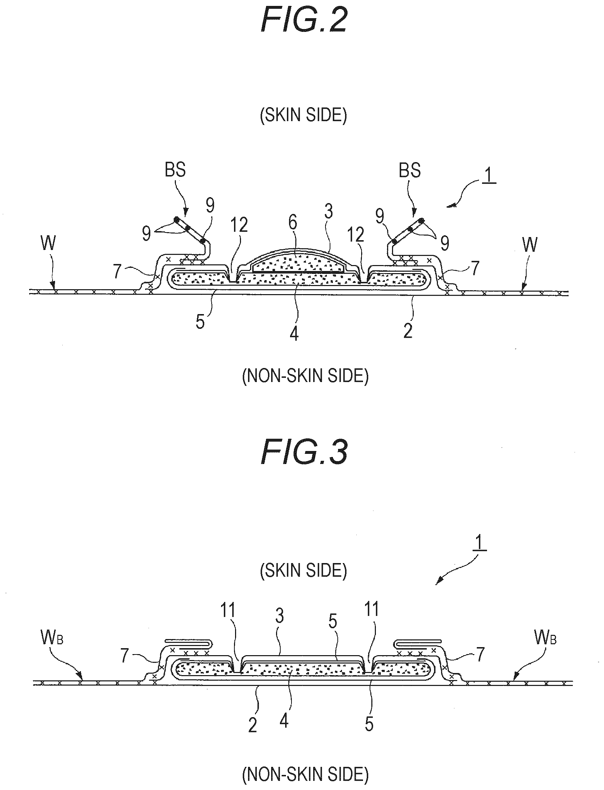

On the skin contact surface side of the absorber 4, a middle-height portion 6 of the absorber which is elongated in the longitudinal direction of the napkin and has an increased thickness toward the skin is provided in a widthwise central portion which is a site including a region corresponding to the bodily fluid discharge part H of the wearer. The middle-height portion 6 is provided to cover a site roughly from the bodily fluid discharge part H of the wearer to a start position of a gluteal region groove, and is provided in a longitudinal section from the wing-shaped flap W to a middle portion of the rear flap W_{B}. The middle-height portion 6 may be formed by integral accumulation with the absorber 4, or may be obtained by stacking separately accumulated ones. When a thickness of the middle-height portion 6 is excessively large, rigidity of the absorber 4 increases and adhesion to the body decreases, and thus the thickness is preferably set to 3 to 20 mm, preferably 5 to 15 mm. Note that when the absorber 4 is surrounded by the encapsulation sheet 5, the absorber 4 and the middle-height portion 6 may be integrally encased as illustrated in Fig. 2, or the absorber 4 and the middle-height portion 6 may be separately surrounded by the encapsulation sheet and then laminated although not illustrated.

Meanwhile, in the illustrated example, the width dimension of the liquid-permeable face sheet 3 is set to be slightly longer than the width dimension of the absorber 4 as illustrated in the cross-sectional views of Figs. 2 and 3 to merely cover the absorber 4. On an outer side thereof, the side nonwoven fabric 7 different from the liquid-permeable face sheet 3, specifically, the side nonwoven fabric 7 made of a nonwoven fabric material subjected to an appropriate water repellent treatment or hydrophilic treatment for the purpose of preventing permeation of menstrual blood, vaginal discharge, etc. or enhancing the feel of the skin is arranged.

As the side nonwoven fabric 7, it is possible to use a side nonwoven fabric formed by an appropriate processing method using natural fiber, synthetic fiber, regenerated fiber, etc. as a material. However, it is preferable to use a nonwoven fabric having air permeability by reducing a basis weight in order to eliminate stickiness and prevent stuffiness. Specifically, it is desirable to use a nonwoven fabric produced to have a basis weight of 13 to 23 g/m², and water-repellent nonwoven fabric coated with a silicon-based, paraffin-based, or alkylchromic chloride-based water repellent is preferably used to prevent permeation of bodily fluid.

In the side nonwoven fabric 7, as illustrated in Figs. 2 and 3, an outer side portion from a widthwise middle portion is bonded by an adhesive such as hot melt over a range from a predetermined inner position to an outer edge of the liquid-impermeable back sheet 2, and a predetermined flap portion is formed at a predetermined position. This flap portion forms the pair of right and left wing-shaped flaps W and W on both sides of a portion substantially corresponding to the bodily fluid discharge part H, and forms the rear flaps W_{B} and W_{B} on both sides of a rear portion on the rear side (gluteal region side) thereof. An adhesive layer (not illustrated) is provided on the outer surface side (liquid-impermeable back sheet 2 side) of each of the wing-shaped flaps W and W and the rear flaps W_{B} and W_{B}. During wearing on shorts, the wing-shaped flaps W and W are folded back to the opposite side at a folding line RL position at a base portion, wound around a crotch part of the shorts, and fixed, and the rear flap W_{B} is fastened to an inner surface of the shorts.

Meanwhile, an inner side portion of the side nonwoven fabric 7 is folded back substantially doubly. Inside this double sheet, one or a plurality of (three in the illustrated example) thread-like elastically stretchable members 9, 9 ... whose both ends or appropriate position in the longitudinal direction is fixed is arranged in a middle portion in a height direction in a state in which the both ends or appropriate position in the longitudinal direction is fixed. At front and rear end portions, as illustrated in Fig. 3, this double sheet part is bonded to the absorber 4 side while being folded back to the outside once, so that the pair of right and left linear three-dimensional gathers BS and BS standing to the skin side while being inclined outward is formed in a predetermined section in the longitudinal direction of the napkin including at least the bodily fluid discharge part H as illustrated in Fig. 2.

### [Compressed groove]

In the sanitary napkin 1, compressed grooves recessed from the skin side of the liquid-permeable face sheet 3 toward the non-skin side are formed in a predetermined region. The compressed grooves are formed by being integrally recessed to the non-skin side from the liquid-permeable face sheet 3 to the absorber 4 by compression from the outer surface side of the liquid-permeable face sheet 3 in a state in which the liquid-permeable face sheet 3 is laminated on a skin-side surface of the absorber 4. The compressed grooves are formed in a widthwise central region not reaching the side nonwoven fabrics 7 and 7 on both sides within a widthwise range in which the liquid-permeable face sheet 3 between the side nonwoven fabrics 7 and 7 on both sides is exposed to the skin side. Hereinafter, the compressed grooves will be described in detail.

In the sanitary napkin 1, in the region on the rear side of the bodily fluid discharge part H of the wearer, a plurality of widthwise compressed grooves 10, 10 ... which extend substantially along the width direction of the sanitary napkin 1, are formed in shapes bulging toward the rear side, and are arranged at intervals in the longitudinal direction of the sanitary napkin 1, and a pair of right and left longitudinally compressed grooves 11, 11 ... which extend substantially along the longitudinal direction of the sanitary napkin 1, and are arranged on the respective outer sides in the width direction at a predetermined separating distance from the widthwise compressed grooves 10 ... are provided.

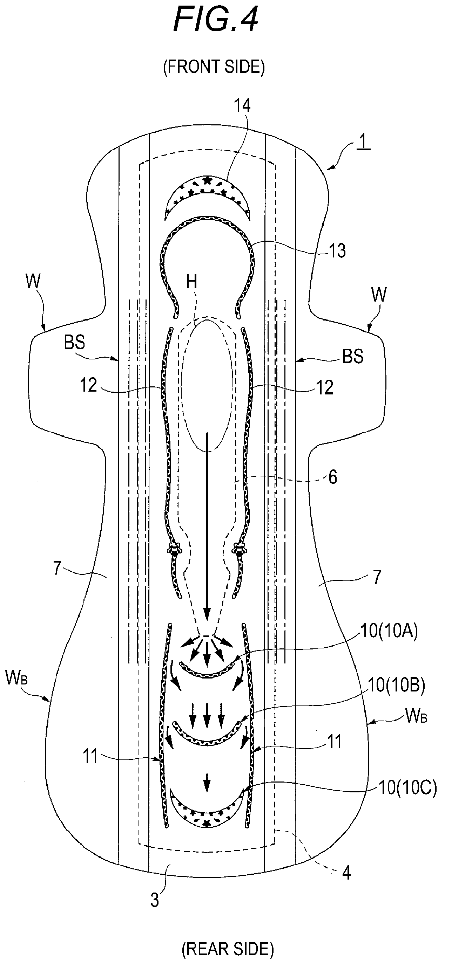

The sanitary napkin 1 has the following effects by providing the widthwise compressed grooves 10 ... and the longitudinally compressed grooves 11 .... Since the widthwise compressed grooves are formed in shapes bulging toward the rear side and the plurality of widthwise compressed grooves is arranged at intervals in the longitudinal direction of the sanitary napkin 1, the bodily fluid that diffuses toward the rear side can be blocked in stages by the plurality of widthwise compressed grooves 10, 10 ... and absorbed by the absorber 4 as illustrated in Fig. 4. Therefore, absorption efficiency of the bodily fluid can be improved, and rear leakage can be prevented.

In addition, since the longitudinally compressed grooves 11 and 11 are arranged on the respective outer sides in the width direction at the predetermined separating distance from the widthwise compressed groove 10, the bodily fluid blocked by the widthwise compressed groove 10 can be prevented from diffusing outward in the width direction and leaking laterally. Moreover, since a separation portion separated in the width direction is provided between the widthwise compressed groove 10 and the longitudinally compressed groove 11, the bodily fluid blocked by the widthwise compressed groove 10 and saturated in this region diffuses to the rear side through the separation portion, so that the bodily fluid can be further absorbed into the rear side region. Therefore, the rear leakage can be reliably prevented.

The widthwise compressed groove 10 is formed in a region on the rear side of the middle-height portion 6 and in the widthwise central portion in a longitudinal section in which the rear flaps W_{B} and W_{B} are formed on both sides. The widthwise compressed groove 10 extends substantially in the width direction of the sanitary napkin 1 and is formed in a bilaterally symmetrical shape with respect to a longitudinal centerline CL of the sanitary napkin 1. That is, a straight line connecting both ends of the widthwise compressed groove 10 is formed in a shape that substantially coincides with a width direction line orthogonal to the longitudinal centerline CL of the sanitary napkin 1.

A plurality of the widthwise compressed grooves 10 is arranged at intervals in the longitudinal direction of the sanitary napkin 1, preferably 2 to 5 pieces, and in the illustrated example, 3 pieces of 10A to 10C are arranged in order from the front side. By arranging the plurality of widthwise compressed grooves 10, it is possible to block the bodily fluid flowing backward in stages, and to reliably prevent the bodily fluid from leaking in the rear portion.

A pitch of the widthwise compressed grooves 10 and 10 (a length of the sanitary napkin 1 in the longitudinal direction between end edges of the widthwise compressed grooves 10 and 10 adjacent to each other) is optional. However, as illustrated in Fig. 1, it is preferable that a pitch P_{BC} between two widthwise compressed grooves 10B and 10C on the rear side is larger than a pitch P_{AB} between two widthwise compressed grooves 10A and 10B on the front side (P_{AB} < P_{BC}). Since the amount of bodily fluid is larger in a part closer to the front side, by decreasing the pitch, the bodily fluid blocked by the widthwise compressed groove 10 can be temporarily stored easily, so that the absorber can rapidly hold the bodily fluid. The pitch P_{AB} between the widthwise compressed grooves 10A and 10B on the front side is preferably 30 to 40 mm, and the pitch P_{BC} between the widthwise compressed grooves 10B and 10C on the rear side is preferably 35 to 45 mm. A difference between these pitches (P_{BC} - P_{AB}) is preferably 5 to 15 mm.

A planar shape of the widthwise compressed groove 10 is preferably a shape bulging rearward to such an extent that an uncompressed region is formed between a straight line connecting both ends of the widthwise compressed groove 10 and the widthwise compressed groove 10, and can be formed by an arc, an elliptic arc, a crescent shape, a polygonal line, etc. bulging toward the rear side of the sanitary napkin 1. It is unnecessary to form all the plurality of widthwise compressed grooves 10, 10 ... arranged at intervals in the longitudinal direction of the sanitary napkin 1 in the same shape, and the widthwise compressed grooves 10, 10 ... may be formed in different shapes such that the two widthwise compressed grooves 10A and 10B on the front side are formed in arc shapes and the rearmost one 10C is formed in a crescent shape as in the illustrated example.

As illustrated in Fig. 1, it is preferable to form the shapes so that lengths M_{A} to Mc along the longitudinal direction of the sanitary napkin 1 from straight lines connecting both ends of the respective widthwise compressed grooves 10A to 10C to rear ends of the widthwise compressed grooves 10A to 10C gradually increase toward the rear side. That is, it is preferable that Ma < M_{B} < Mc. In this way, since more bodily fluid diffuses from the front side in the widthwise compressed groove 10 arranged on the front side, the amount of bodily fluid that can be blocked and temporarily stored by the widthwise compressed groove 10 is reduced, and more bodily fluid is allowed to flow to the rear side through the separation portion between the widthwise compressed groove 10 and the longitudinally compressed groove 11 on both sides, so that the bodily fluid blocked in this region is prevented from leaking. Further, the widthwise compressed groove 10 arranged on the rear side is formed in a shape that can block more bodily fluid to prevent leakage from the rear end. The length M_{A} of the widthwise compressed groove 10A at a forefront portion is preferably 7 to 12 mm, the length M_{B} of the widthwise compressed groove 10B at the middle portion is preferably 12 to 17 mm, and the length Mc of the widthwise compressed groove 10C at a rearmost portion is preferably 20 to 25 mm. In addition, referring to ratios of the lengths M_{B} and Mc to the length M_{A} of the widthwise compressed groove 10A at the forefront portion, M_{B} is preferably set to 1.2 to 1.7, and Mc is preferably set to 2.0 to 2.5.

It is preferable that a groove width of the widthwise compressed groove 10 gradually increases so that one arranged relatively on the rear side of the sanitary napkin 1 has a larger groove width. In the compressed groove, when the groove width is increased, it becomes more difficult for the bodily fluid to pass through the groove, and thus the effect of blocking the bodily fluid is enhanced. Therefore, the widthwise compressed groove 10 arranged on the rear side has a higher effect of blocking the bodily fluid, and rear leakage of the bodily fluid can be more reliably prevented. The groove width is a length between both side walls orthogonal to the centerline in the groove longitudinal direction. In a widthwise compressed groove formed to have a substantially equal width over the entire length such as the two widthwise compressed grooves 10A and 10B, it is possible to use a groove width at an arbitrary intermediate position. In a widthwise compressed groove formed in a crescent shape to have a groove width changing depending on the location such as the widthwise compressed groove 10C at the rearmost portion, it is possible to use an average groove width over the entire length. The groove width of the widthwise compressed groove 10A at the forefront portion is preferably 1.0 to 3.0 mm, the groove width of the widthwise compressed groove 10B at the middle portion is preferably 1.5 to 3.5 mm, and the groove width of the widthwise compressed groove 10C at the rearmost portion is preferably 5.0 to 9.0 mm. In addition, referring to a ratio of the groove width of the widthwise compressed groove 10B at the middle portion and a ratio of the groove width of the widthwise compressed groove 10C at the rearmost portion to the groove width of the widthwise compressed groove 10A at the forefront portion, it is preferable that the ratio of the groove width of the widthwise compressed groove 10B at the middle portion is set to 1.1 to 1.4 and the ratio of the groove width at the rearmost portion is set to 3.0 to 4.0.

It is preferable that the length of the widthwise compressed groove 10 along the width direction of the sanitary napkin 1 gradually increases so that one arranged relatively on the rear side of the sanitary napkin 1 has a larger length. That is, it is preferable that the length L_{A} of the widthwise compressed groove 10A at the forefront portion, the length L_{B} of the widthwise compressed groove 10B at the middle portion, and the length Lc of the widthwise compressed groove 10C at the rearmost portion have a relationship of L_{A} < L_{B} < L_{C}. Since it is possible to block more bodily fluid flowing to the rear side by increasing the length along the width direction of the napkin, the widthwise compressed groove 10 arranged on the rear side is more effective in blocking bodily fluid, and the rear leakage of the bodily fluid can be more reliably prevented. The length L_{A} of the widthwise compressed groove 10A at the forefront portion is preferably 30 to 35 mm, the length L_{B} of the widthwise compressed groove 10B at the middle portion is preferably 37 to 42 mm, and the length Lc of the widthwise compressed groove 10C at the rearmost portion is preferably 40 to 45 mm. In addition, referring to a ratio of the length L_{B} of the widthwise compressed groove 10B at the middle portion and a ratio of the length Lc of the widthwise compressed groove 10C at the rearmost portion to the length L_{A} of the widthwise compressed groove 10A at the forefront portion, it is preferable that the ratio of the length L_{B} of the widthwise compressed groove 10B at the middle portion is set to 1.1 to 1.4 and the ratio of the length Lc at the rearmost portion is set to 1.2 to 1.5.

Next, the longitudinally compressed groove 11 is formed to continuously extend along the substantially longitudinal direction of the sanitary napkin 1 within a longitudinal section in which the rear flaps W_{B} and W_{B} are formed on both sides and in a longitudinal section including a section in which the widthwise compressed grooves 10, 10 ... are formed. In the longitudinally compressed groove 11, a straight line connecting a front end and a rear end extends along the substantially longitudinal direction of the sanitary napkin 1, and an angle between this straight line and the longitudinal centerline CL of the sanitary napkin 1 is preferably formed within a range of 0 to ±45°. In this way, the bodily fluid is inhibited from diffusing outward in the width direction from the longitudinally compressed groove 11, and lateral leakage at the rear can be reliably prevented.

The longitudinally compressed grooves 11 are arranged on both sides in the width direction at a predetermined separating distance from side edges of the widthwise compressed groove 10, and are formed symmetrically with respect to the longitudinal centerline CL of the sanitary napkin 1. That is, the longitudinally compressed groove 11 is not connected to the widthwise compressed groove 10, and a non-compressed portion that is not compressed is interposed between the longitudinally compressed groove 11 and the widthwise compressed groove 10. In this way, without the bodily fluid staying in a region surrounded by the widthwise compressed groove 10 and the longitudinally compressed grooves 11 and 11 on both sides, the bodily fluid can diffuse to the rear side through the non-compressed portion between the widthwise compressed groove 10 and the longitudinally compressed groove 11. For this reason, it is possible to prevent leakage caused by the bodily fluid staying in a predetermined region overflowing over the compression groove.

The separating distance between the widthwise compressed groove 10 and the longitudinally compressed groove 11 along the width direction of the sanitary napkin 1 is arbitrary. However, as illustrated in Fig. 1, it is preferable that the separating distance gradually decreases so that one arranged relatively on the rear side has a smaller separating distance. That is, when a separating distance between the widthwise compressed groove 10A at the forefront portion and the longitudinally compressed groove 11 is set to S_{A}, a separating distance between the widthwise compressed groove 10B at the middle portion and the longitudinally compressed groove 11 is set to S_{B}, and a separating distance between the widthwise compressed groove 10C at the rearmost portion and the longitudinally compressed groove 11 is set to Sc, it is preferable to set S_{A} > S_{B} > S_{C}. In this way, the amount of bodily fluid that diffuses to the rear side through the non-compressed portion in this separation part gradually decreases, and leakage from the rear side can be more reliably prevented. The separating distance can be arbitrarily set within a range of 2.0 to 10.0 mm. Further, a ratio of the separating distance S_{B} of the middle portion to the separating distance S_{A} of the forefront portion and a ratio the separating distance Sc of the rearmost portion to the separating distance Sa of the forefront portion are preferably set to 0.72 to 0.77 and 0.40 to 0.45, respectively.

A planar shape of the longitudinally compressed groove 11 is arbitrary. However, as illustrated in Fig. 1, the planar shape is preferably an arc or an elliptic arc that bulges outward in the width direction of the sanitary napkin 1. By forming the planar shape to bulge outward in the width direction, bodily fluid can be easily retained inside the longitudinally compressed groove 11, and leakage from the side portion can be prevented more reliably.

The groove width of the longitudinally compressed groove 11 may be equal to or less than the groove width of the widthwise compressed groove 10 or larger than the groove width of the widthwise compressed groove 10. Preferably, in order to prevent leakage from the side more reliably, it is preferable to form the groove width approximately equal to the groove width of the widthwise compressed groove 10B in the middle portion.

The longitudinally compressed groove 11 extends to a position where the front end overlaps a rear end portion of the middle-height portion 6 in the width direction. That is, a rear end of the middle-height portion 6 is located on the rear side of a front end of the longitudinally compressed groove 11. The longitudinally compressed grooves 11 are arranged at positions separated from the middle-height portion 6 to the outside in the width direction. In this way, the bodily fluid absorbed into the middle-height portion 6 is diffused from the rear end of the middle-height portion 6 to the regions where the longitudinally compressed grooves 11 and 11 are arranged on both sides, and thus the bodily fluid is reliably into the absorber 2 by the longitudinally compressed grooves 11 and 11 and the widthwise compressed groove 10. As illustrated in Fig. 1, a length U of an overlapping portion of the middle-height portion 6 and the longitudinally compressed groove 11 in the napkin width direction is preferably set to 5.0 to 10.0 mm, and more preferably 7.0 to 8.0 mm so that the bodily fluid can efficiently diffuse from the middle-height portion 6 into a region interposed between the longitudinally compressed grooves 11 and 11.

As illustrated in Fig. 1, in the sanitary napkin 1, a pair of left and central longitudinally compressed grooves 12 and 12 extending along substantially the longitudinal direction of the sanitary napkin 1 is provided on both sides of a region including a region corresponding to the bodily fluid discharge part H of the wearer. The central longitudinally compressed grooves 12 and 12 are formed on both sides of the middle-height portion 6 so as to extend substantially along side edges of the middle-height portion 6. A front end of the central longitudinally compressed groove 12 is provided at the same position as that of the front end of the middle-height portion 6, and a rear end thereof is arranged on a front side of the rear end of the middle-height portion 6 and at a position apart from the front ends of the longitudinally compressed grooves 11 and 11 to the front side. Note that the rear end of the central longitudinally compressed groove 12 may be arranged at a position which substantially coincides with the front end of the longitudinally compressed groove 11 in the longitudinal direction of the napkin or at the rear side thereof. In this case, rear portions of the central longitudinally compressed grooves 12 and 12 are preferably provided at positions separated from the longitudinally compressed grooves 11 and 11 to the central side in the napkin width direction.

A right-left separating distance X₁₂ at the rear end of the central longitudinally compressed groove 12 is preferably smaller than a right-left separating distance X₁₁ at the front end of the longitudinally compressed groove 11 (X₁₂ < X₁₁). In this way, the bodily fluid diffused to the rear side along the central longitudinally compressed groove 12 easily diffuses to the region interposed between the longitudinally compressed grooves 11 and 11, the bodily fluid is reliably absorbed into the absorber 4 by the longitudinally compressed grooves 11 and 11 and the widthwise compressed groove 10, and leakage from the side can be prevented. A difference (X₁₁ - X₁₂) in the separating distance is preferably set to 10.0 to 15.0 mm.

It is preferable that the rear end of the central longitudinally compressed groove 12 is bent toward the longitudinal centerline CL side of the sanitary napkin 1, and a straight line extending from the rear end of the central longitudinally compressed groove 12 is formed to extend toward the right and left separation portions at the front ends of the longitudinally compressed grooves 11 and 11. In this way, the bodily fluid diffusing to the rear side along the central longitudinally compressed groove 12 easily diffuses to the region interposed between the longitudinally compressed grooves 11 and 11.

In the sanitary napkin 1 illustrated in Fig. 1, in order to prevent leakage from the front side, a first front side compressed groove 13 having a substantially Ω shape in plan view is formed apart from the front side of the central longitudinally compressed groove 12, and a second front side compressed groove 14 having a substantially crescent shape in plan view is formed apart from a front side of the first front side compressed groove 13.

### [Other embodiments]

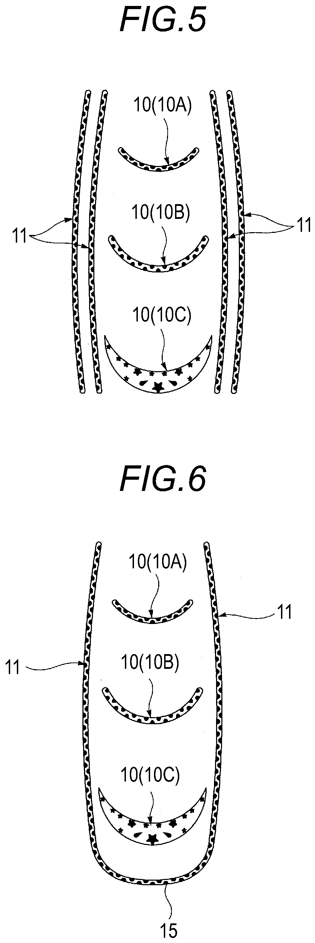

(1) In the above embodiment, one longitudinally compressed groove 11 is provided on each of the right and left sides. However, as illustrated in Fig. 5, a plurality of longitudinally compressed grooves 11 (two longitudinally compressed grooves 11 in the illustrated example) may be provided on each of the right and left sides. By providing the plurality of longitudinally compressed grooves 11 on each of the sides, it is possible to reliably prevent leakage from the side portion at the rear side.
(2) In the above embodiment, the rear ends of the right and left longitudinally compressed grooves 11 and 11 are formed separated from each other to the right and left. However, as illustrated in Fig. 6, by providing a connecting compressed groove 15 that connects the rear ends of the right and left longitudinally compressed grooves 11 and 11 to each other, the rear ends of the right and left longitudinally compressed grooves 11 and 11 may be connected to each other. By connecting the rear ends of the right and left longitudinally compressed grooves 11 and 11 to each other, the bodily fluid can be absorbed and held in a region surrounded by the longitudinally compressed grooves 11 and 11 and the connecting compressed groove 15, and thus the rear leakage of the bodily fluid can be more reliably prevented.

### Reference Signs List

- 1: Sanitary napkin
- 2: Liquid-impermeable back sheet
- 3: Liquid-permeable face sheet
- 4: Absorber
- 5: Encapsulation sheet
- 6: Middle-height portion
- 7: Side nonwoven fabric
- 9: Thread-like elastically stretchable member
- 10, 10A, 10B, 10C: Widthwise compressed groove
- 11: Longitudinally compressed groove
- 12: Central longitudinally compressed groove

## Claims

1. An absorbent article (1) in which an absorber (4) is interposed between a face sheet (3) and a back sheet (2), the absorbent article comprising:
a plurality of widthwise compressed grooves (10) which is disposed in a region on a rear side of a region corresponding to a bodily fluid discharge part of a wearer, extends along substantially a width direction of the absorbent article, is formed in shapes bulging toward a rear side, and is arranged at intervals in a longitudinal direction of the absorbent article, wherein the groove widths gradually increase so that one arranged relatively on the rear side of the absorbent article has a larger groove width; and
a pair of right and left longitudinally compressed grooves (11) which extends along substantially a longitudinal direction of the absorbent article and is arranged on outer sides in the width direction at a predetermined separating distance from the widthwise compressed grooves, respectively, wherein separating distances between the widthwise compressed grooves and the longitudinally compressed grooves gradually decrease so that one arranged relatively on the rear side of the absorbent article has a smaller separating distance.

2. The absorbent article according to claim 1, wherein lengths of the widthwise compressed grooves gradually increase so that one arranged relatively on the rear side of the absorbent article has a larger length.

3. The absorbent article according to any one of claims 1 or 2, wherein the longitudinally compressed grooves are formed in shapes bulging outward in the width direction of the absorbent article.

4. The absorbent article according to any one of claims 1 to 3, wherein a middle-height portion of the absorber thickened toward a skin side is formed in a section including the region corresponding to the bodily fluid discharge part of the wearer, and a rear end of the middle-height portion is located on a rear side of front ends of the longitudinally compressed grooves.

5. The absorbent article according to any one of claims 1 to 4, wherein a pair of right and left central longitudinally compressed grooves extending along substantially the longitudinal direction of the absorbent article is provided on both sides of a region including the region corresponding to the bodily fluid discharge part of the wearer, and a right-left separating distance at rear ends of the central longitudinally compressed grooves is smaller than a right-left separating distance at front ends of the longitudinally compressed grooves.

## Patentansprüche

1. Absorbierender Artikel (1), in dem ein Absorber (4) zwischen eine vordere Folie (3) und eine hintere Folie (2) eingefügt ist, wobei der absorbierende Artikel Folgendes umfasst:
eine Vielzahl von der Breite nach komprimierten Nuten (10), die in einer Region auf einer hinteren Seite einer Region angeordnet ist, die einem Körperfluid-Austrittsteil eines Trägers entspricht, der sich im Wesentlichen in einer Breitenrichtung des absorbierenden Artikels erstreckt, in Formen gebildet ist, die sich hin zu einer hinteren Seite ausbuchten und in Interwallen in einer Längsrichtung des absorbierenden Artikels angeordnet ist, wobei die Nutenbreiten allmählich zunehmen, so dass eine, die relativ auf der hinteren Seite des absorbierenden Artikels angeordnet ist, eine größere Nutenbreite aufweist; und
ein Paar rechter und linker der Länge nach komprimierter Nuten (11), das sich im Wesentlichen in einer Längsrichtung des absorbierenden Artikels erstreckt und auf Außenseiten in der Breitenrichtung in einem vorbestimmten Trennabstand jeweils von den der Breite nach komprimierten Nuten angeordnet ist, wobei die Trennabstände zwischen den der Breite nach komprimierten Nuten und den der Länge nach komprimierten Nuten allmählich abnehmen, so dass eine, die relativ auf der hinteren Seite des absorbierenden Artikels angeordnet ist, einen kleineren Trennabstand aufweist.

2. Absorbierender Artikel nach Anspruch 1 wobei Längen der der Breite nach komprimierten Nuten allmählich zunehmen, so dass eine, die relativ auf der hinteren Seite des absorbierenden Artikels angeordnet ist, eine größere Länge aufweist.

3. Absorbierender Artikel nach einem der Ansprüche 1 oder 2, wobei die der Länge nach komprimierten Nuten in Formen gebildet sind, die sich nach außen in der Breitenrichtung des absorbierenden Artikels ausbuchten.

4. Absorbierender Artikel nach einem der Ansprüche 1 bis 3, wobei ein mittelhoher Abschnitt des Absorbers, der hin zu einer auf die Haut gerichteten Seite verdickt ist, in einem Abschnitt gebildet ist, der die Region beinhaltet, die dem Körperfluid-Austrittsteil des Trägers entspricht, und sich ein hinteres Ende des mittelhohen Abschnitts auf einer hinteren Seite der vorderen Enden der der Länge nach komprimierten Nuten befindet.

5. Absorbierender Artikel nach einem der Ansprüche 1 bis 4, wobei ein Paar rechter und linker zentraler der Länge nach komprimierter Nuten, die sich im Wesentlichen in einer Längsrichtung des absorbierenden Artikels erstrecken, auf beiden Seiten einer Region bereitgestellt ist, darin eingeschlossen die Region, die dem Körperfluid-Austrittsteil des Trägers entspricht, und ein Rechts-links-Trennabstand an hinteren Enden der der Länge nach komprimierten Nuten kleiner als ein Rechts-links- Trennabstand an vorderen Enden der der Länge nach komprimierten Nuten ist.

## Revendications

1. Article absorbant (1) dans lequel un absorbeur (4) est intercalé entre une feuille de dessus (3) et une feuille de dessous (2), l'article absorbant comprenant :
une pluralité de rainures comprimées dans le sens de la largeur (10) qui est disposée dans une région sur un côté arrière d'une région correspondant à une partie de décharge de fluide corporel d'un utilisateur, s'étend sensiblement le long d'une direction de largeur de l'article absorbant, est formée dans des formes se renflant vers un côté arrière et est agencée à intervalles dans une direction longitudinale de l'article absorbant, dans lequel les largeurs de rainure augmentent progressivement de sorte que celle agencée relativement sur le côté arrière de l'article absorbant a une plus grande largeur de rainure ; et
une paire de rainures droite et gauche longitudinalement comprimées (11) qui s'étend sensiblement le long d'une direction longitudinale de l'article absorbant et est agencée sur les côtés externes dans la direction de largeur à une distance de séparation prédéterminée des rainures comprimées dans le sens de la largeur, respectivement, dans lequel les distances de séparation entre les rainures comprimées dans le sens de la largeur et les rainures comprimées longitudinalement diminuent progressivement de sorte que celle agencée relativement sur le côté arrière de l'article absorbant a une plus petite distance de séparation.

2. Article absorbant selon la revendication 1, dans lequel les longueurs des rainures comprimées dans le sens de la largeur augmentent progressivement de sorte que celle agencée relativement sur le côté arrière de l'article absorbant a une plus grande longueur.

3. Article absorbant selon l'une quelconque des revendications 1 ou 2, dans lequel les rainures comprimées longitudinalement sont formées selon des formes se renflant vers l'extérieur dans la direction de largeur de l'article absorbant.

4. Article absorbant selon l'une quelconque des revendications 1 à 3, dans lequel une partie de hauteur moyenne de l'absorbeur épaissie vers un côté de peau est formée dans une section comprenant la région correspondant à la partie de décharge de fluide corporel de l'utilisateur, et une extrémité arrière de la partie de hauteur moyenne est positionnée sur un côté arrière des extrémités avant des rainures longitudinalement comprimées.

5. Article absorbant selon l'une quelconque des revendications 1 à 4, dans lequel une paire de rainures droite et gauche longitudinalement comprimées s'étendant sensiblement le long de la direction longitudinale de l'article absorbant est prévue des deux côtés d'une région comprenant la région correspondant à la partie de décharge de fluide corporel de l'utilisateur, et une distance de séparation droite-gauche au niveau des extrémités arrière des rainures centrales longitudinalement comprimées est inférieure à une distance droite-gauche au niveau des extrémités avant des rainures longitudinalement comprimées.
